# EUROPEAN PATENT APPLICATION

(11) **EP 1 520 515 A1**
(43) Date of publication of application: **06.04.2005**
(21) Application number: 04103770.6
(22) Date of filing: 05.08.2004
(51) Int. Cl.: A61B 5/103

(54) **Heat algometer**

(30) Priority: 05.09.2003 IT GE20030068
(71) Applicant: R.G.M. S.p.A., 16153 Genova (IT)
(72) Inventor: Gallamini, Michele, 16167, GENOVA (IT); Calza', Laura, 40050, MONTERENZIO, Province of Bologna (IT); Capra, Roberto, 17100, SAVONA (IT); Cornaglia Ferraris, Paolo, 16167, GENOVA (IT); Cartabianca, Luca, 16152, GENOVA (IT)
(74) Representative: Porsia, Attilio, Dr.

(57) **Abstract**

Portable apparatus for evaluating pain by processing measurements of the sensitivity and thermal pain thresholds, comprising: means (1) for cooling and heating a portion of the patient's skin; temperature detection means (1, 101) associated with said heating and cooling means (1); a central processing unit (2) which is connected to said heating and cooling means (1) and to said temperature detection means (1, 101) and is provided with a memory unit (202) and means for displaying the data detected (102); and power supply means

## Description

The present invention relates to an apparatus for evaluating pain by processing measurements of the sensitivity and thermal pain thresholds; such an apparatus may be more simply referred to by the single expression: "heat algometer".

In view of the mechanisms associated with pain and the complex nature of its emotional experience, it is perhaps impossible to propose systems for performing objective, repeatable and comparable measurements. Clinical practice uses subjective scales which essentially require the patient to express his/her perception in analog form (VAS), in numerical form by means of a pain rating (NRS) or in graphical form by making a specific selection from a scale of illustrations representing increasing levels of pain (FPS). The data is fairly stable and reliable, although it is obviously influenced greatly by the way in which the question regarding identification of one's particular state is posed.

If we analyse briefly the macroscopic pain perception mechanism it can be observed that the transduction, transmission, modulation and perception phenomena interact with the reaction of the system in a manner which can be simplified as follows. The peripheral stimulus, which is modulated by the spinothalamic reactions, reaches the cortical system which issues commands for action which, even though modulated by the spinothalamic system, produce a muscular reaction. It should be noted moreover that, even in the absence of cortical intervention, reactions via the spinothalamic system may occur.

On the basis of the above observations, the possibility of developing an analytical approach, i.e. an approach which is as objective as possible, with regard to reactions of the system to particular types of stimulus was examined.

Already in 1983 the problem of peripheral thermal stimulation was considered and the conclusion reached that the quality of the pain generated was influenced in a decisive manner both by the duration of the stimulus and the area to which the stimulus was applied (Chery-Croze S.; "Painful sensation induced by a thermal cutaneous stimulus" - Pain, 1983, Oct. 17(2): 109-37). Subsequently, a significant variability of the sensitivity thresholds in various zones of the body was established, together with a limited and moderate variability in the data detected for the same patient (Meh D., Denislic M.; "Quantitative assessment of thermal and pain sensitivity - J. Neurol. Sci. 1994 Dec. 20; 127(2): 164-9). The zone which was most reliable during measurement of the thermal sensorial thresholds is the hand and in particular more interesting results were obtained for the cold perception threshold recorded on the ball of the thumb (Louise G. Hagander, Hani A. Midani, Michael A. Kuskowski, Gareth J.G. Perry; "Quantitative sensory testing: effect of site and skin temperature on thermal thresholds - Clin. Neurophysiology 111 (2000) 17-22).

Despite numerous further studies relating to the use of the thermal sensitivity data in order to obtain significant information for determining the pain threshold, hitherto a complete and simply operated apparatus for monitoring patients in this specific area has not been developed.

The object of the present invention is therefore that of providing an apparatus which aims to produce an objective measurement which can be correlated to the state of suffering of the patient; evaluation must be made possible by means of a measurement cycle which is rapid and devoid of negative effects and the data obtained may be used for clinical and therapeutic purposes.

The present invention therefore relates to a portable apparatus for evaluating pain by processing measurements of the sensitivity and thermal pain thresholds, comprising: means for cooling and heating a portion of the patient's skin; temperature detection means associated with said heating and cooling means; a central processing unit which is connected to said heating and cooling means and to said temperature detection means and is provided with a memory unit and means for displaying the data detected.

The present invention also relates to a method for determining the thermal sensitivity and thermal pain thresholds, comprising the steps of: cooling the patient's skin; detecting the temperature data corresponding to perception of the cooling sensation by the patient; heating the patient's skin; detecting the temperature data corresponding to perception of the heating sensation; further heating of the patient's skin; detection of the temperature data corresponding to the perception of thermal pain.

Further advantages and characteristic features will emerge from the following detailed description of an embodiment of the apparatus and the method according to the present invention provided by way of a nonlimiting example, with reference to the accompanying drawings in which:
Figure 1 is a schematic diagram illustrating the apparatus according to the present invention; and
Figures 2A and 2B are the two parts of a flow diagram which schematically represents operation of the apparatus according to the method of the present invention.

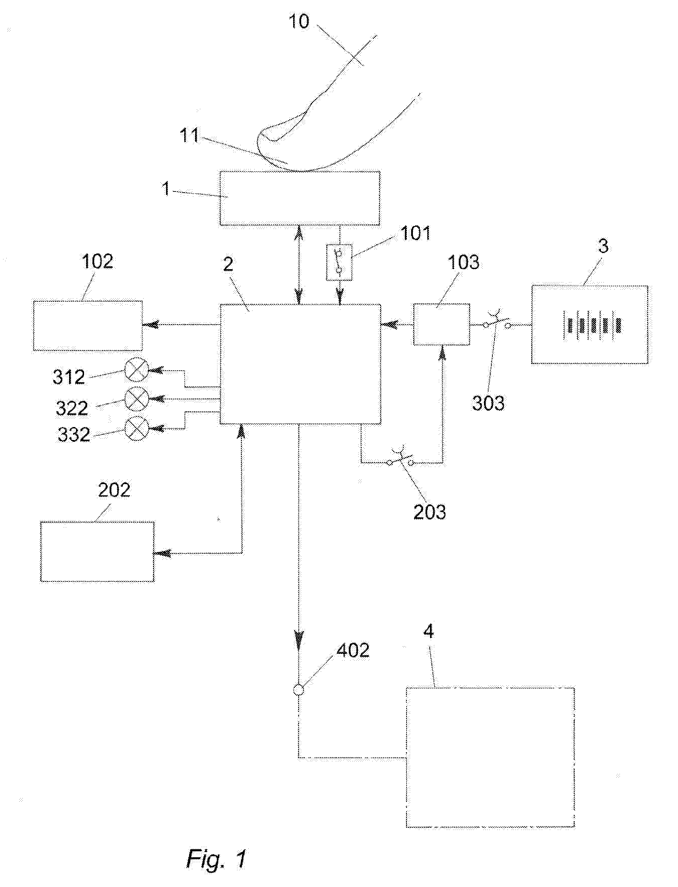

Figure 1 shows schematically the apparatus according to the invention: 1 denotes the heating and cooling and temperature detection means, consisting of a Peltier cell, which is in contact with the tip 11 of the patient's finger 10 and which is connected to the central processing unit 2 and provided with a switch 101. The central unit 2 is provided with a display device 102 and with three luminous signalling means 312, 322 and 332; it also has a memory unit 202 and a data output 402 for transfer, where necessary, of the data acquired to another unit 4. The apparatus is powered by means of the battery 3 which is connected to the central unit by means of the inverter 103; a main switch 303 is inserted along the connection, while the inverter is turn controlled by the switch 203.

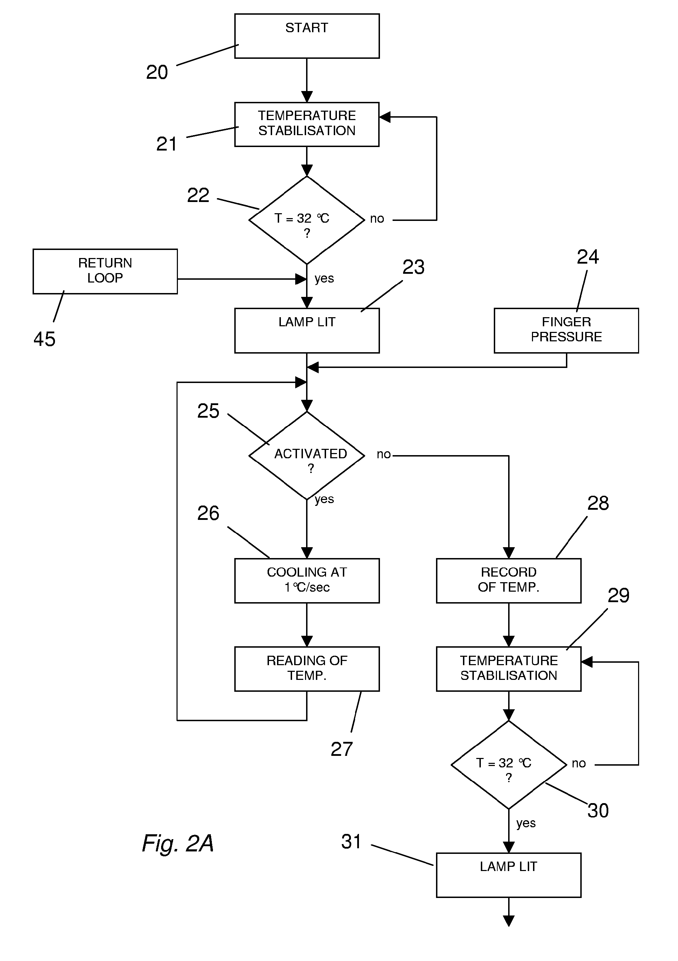

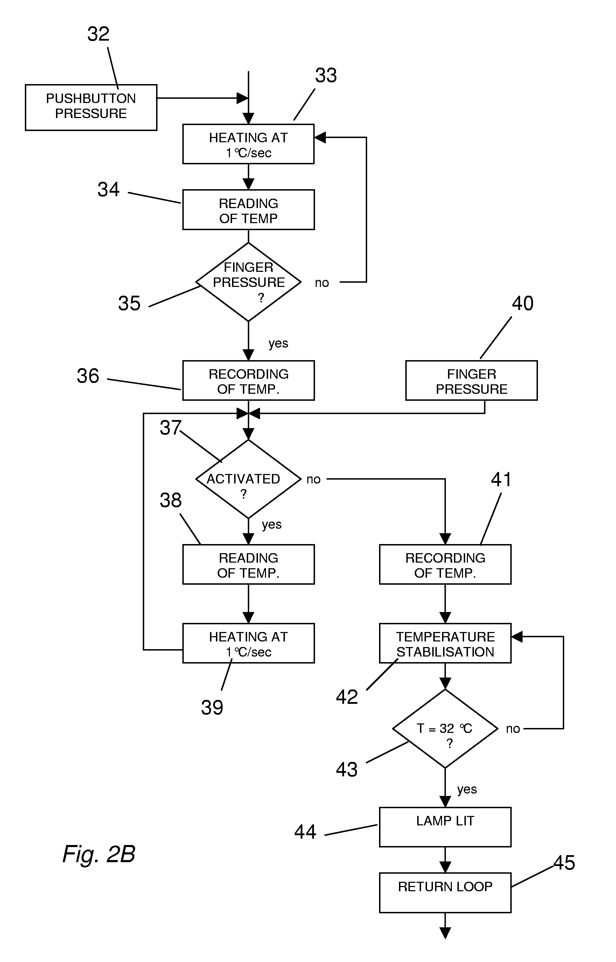

The operating principle of the apparatus according to the present invention will become clear from that described below, with direct reference to the flow diagram illustrated in Figures 2A and 2B. Switching-on (step 20, Fig. 2A), performed by operating the switch 303 and signalled by the luminous lamp 312, is followed by stabilisation of the cell temperature until a temperature of 32°C is reached (steps 21, 22); this temperature is considered to be the optimum temperature for recording data according to the method of the invention; lighting-up of a lamp (23), for example the indicator lamp 322 in the diagram of Figure 1, indicates that the apparatus is ready to operate. Pressure of the fingertip on the cell (24) starts the cooling cycle (25-27) which takes place with a scanning rate of 1°C per second; the patient does not release the pressure on the cell 1 until he/she perceives the sensation of cooling. The pressure applied by the patient is detected by means of the switch 101 associated with the cell, which thus allows activation of the operating sequences programmed in the central unit 2. At that point, the temperature at which the patient perceived the sensation of cooling is recorded (28) and the temperature of the cell is reset to 32°C until stabilisation occurs, indicated again by lighting-up of the indicator lamp 322 (29-31).

When the apparatus is ready to operate again, the switch 203 is operated (step 32, Fig. 2B), starting operation of the inverter 103 and thus allowing the cell 1 to release heat instead of absorbing it as occurred during the previous steps. Heating takes place at a speed of 1°C per second and the patient does not press the cell 1 until he/she perceives the sensation of heating (33-35). Upon application of pressure by the patient (40) the corresponding temperature is recorded, while the cell 1 continues to heat up by 1°C per second (37-39); in this situation, release of the pressure by the patient, which occurs when he/she perceives the sensation of thermal pain, causes recording of the temperature at which the patient perceived pain (41). Thereafter the cell is stabilised again (42-44) at 32°C and the apparatus is again able to record data from other patients (45).

In the apparatus according to the invention a Peltier cell is used both owing its capacity for heat reversal, namely reversal in the polarity of the current produces a change-over from absorption to emission of heat, and owing to its effectiveness in the measurement of temperature. The cell has suitable dimensions for insertion in a portable apparatus and in particular is preferably provided with a plate having dimensions of the order of 30 x 30 mm. Control of the cell, performed by the central unit 2, is conducted under the following conditions:

| | |
|---|---|
| Starting and adaptation temperature: | 32°C |
| Thermal gradient | 1°C/s |
| Upper heating limit: | 60°C |
| Lower cooling limit: | 3°C |

At the end of the operating cycle, the display 102 of the apparatus will show, in degrees and tenths of a degree, the cold sensitivity threshold data, heat sensitivity threshold and thermal pain threshold. This data may be recorded in the memory 202 of the apparatus: optionally the apparatus may be provided with data input means, for example a keyboard, in order to supplement the recorded data with the personal data of the patient. The stored data may be transferred to another unit 4 by means of the communications port 402.

## Claims

1. Portable apparatus for evaluating pain by processing measurements of the sensitivity and thermal pain thresholds, comprising: means (1) for cooling and heating a portion of the patient's skin; temperature detection means (1, 101) associated with said heating and cooling means (1); a central processing unit (2) which is connected to said heating and cooling means (1) and to said temperature detection means (1, 101) and is provided with a memory unit (202) and means (102) for displaying the data detected; and power supply means (3).

2. Apparatus according to Claim 1, in which luminous signalling means able to indicate the operative state of the apparatus (312) and its operating condition (322) are also provided.

3. Apparatus according to Claim 1 or 2, in which said central processing unit (2) is provided with a port (402) for connection to another processing unit (4).

4. Apparatus according to any one of the preceding Claims 1 to 3, in which said cooling and heating means and said temperature detection means comprise a Peltier cell (1).

5. Apparatus according to Claim 4, in which said Peltier cell (1) is controlled by said central unit (2) and is powered by means of an inverter (103) driven by the same, the reversal in the polarity of the power supply being controlled by switch means (203).

6. Apparatus according to Claim 4 or 5, in which said Peltier cell (1) has an adaptation and starting temperature of the order of 32°C.

7. Apparatus according to one of the preceding Claims 4 to 6, in which said Peltier cell (1) is heated or cooled with a thermal gradient of the order of 1°C/s.

8. Apparatus according to one of the preceding Claims 4 to 7, in which the upper heating limit of said Peltier cell (1) is of the order of 60°C.

9. Apparatus according to one of the preceding Claims 4 to 8, in which the lower cooling limit of said Peltier cell (1) is of the order of 3°C.

10. Apparatus according to any one of the preceding Claims 4 to 9, in which the skin portion of the patient on which measurement is performed is the tip (11) of a finger (10) of one hand.

11. Apparatus according to any one of the preceding Claims 4 to 10, in which said cell is provided with a plate with dimensions of about 30 x 30 mm.

12. Apparatus according to Claim 10 or 11, in which said Peltier cell (1) is associated with switching means (101) which detect the application of pressure and release of pressure by the patient's fingertip (11), said pressure or said release causing recording of the temperature data detected.

13. Method for determining the thermal sensitivity and thermal pain thresholds, comprising the steps of: cooling a portion of the patient's skin; detecting the temperature data corresponding to perception of the cooling sensation by the patient; heating the same portion of the patient's skin; detecting the temperature data corresponding to perception of the heating sensation; further heating of the same portion of the patient's skin; detection of the temperature data corresponding to perception of the thermal pain.
